# FASCICULE DE BREVET EUROPEEN

(11) **EP 2 334 622 B1**
(45) Date de publication et mention de la délivrance du brevet: **26.08.2015**
(21) Numéro de dépôt: 09786235.3
(22) Date de dépôt: 11.09.2009
(51) Int. Cl.: C07C 17/04, C07C 17/087, C07C 17/20, C07C 17/25, C07C 21/18, C07C 51/12

(54) **PROCEDE DE PREPARATION DE COMPOSES TRIFLUORES ET TETRAFLUORES**
VERFAHREN ZUR HERSTELLUNG TRIFLUORIERTER UND TETRAFLUORIERTER VERBINDUNGEN
PROCESS FOR THE PREPARATION OF TRIFLUORINATED AND TETRAFLUORINATED COMPOUNDS

(30) Priorité: 11.09.2008 FR 0805000
(43) Date de publication de la demande: 22.06.2011
(73) Titulaire: Arkema France, 92700 Colombes (FR)
(72) Inventeur: PIGAMO, Anne, F-69340 Francheville (FR); DEVIC, Michel, F-69110 Sainte-Foy-Les-Lyon (FR); WENDLINGER, Laurent, F-69510 Soucieu en Jarrest (FR)
(74) Mandataire: Hirsch & Associés
(86) Numéro de dépôt international: PCT/IB2009/006803
(87) Numéro de publication internationale: WO 2010/029419

(56) Documents cités:
- WO-A2-2008/075017
- R.N. HASZELDINE: "Reactions of Fluorocarbon Radicals Part V.* Alternative Syntheses for Trifluoromethylacetylene (3:3:3-Trifluoropropyne), and the Influence of Polyfluoro-groups on Adjacent Hydrogen and Halogen Atoms" JOURNAL OF THE AMERICAN CHEMICAL SOCIETY, 1951, pages 2495-2504, XP002503499
- M. BÜCHNER, A. NIXDORF, H.-F. GRÜTZMACHER: "Reactions of Gaseous, Halogentated Propene Radical Cations with Ammonia: A Study of the Mechanism by Fourier Transform Ion Cyclotron Resonance" CHEMISTRY - A EUROPEAN JOURNAL, vol. 4, 1998, pages 1799-1809, XP002517111

## Description

### DOMAINE DE L'INVENTION

L'invention a pour objet un procédé de préparation de composés fluorés, à savoir le composé 2,3-dichloro-1,1,1-trifluoropropane (243db), à partir duquel peut être fabriqué le composé fluoré 2,3,3,3-tétrafluoro-1-propène (HFO 1234yf).

### ARRIERE-PLAN TECHNOLOGIQUE

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne pose pas de problème pour la couche d'ozone.

On connaît plusieurs procédés de fabrication du 1234yf.

WO2007/079431 décrit la préparation de 1234yf par un procédé comprenant les étapes de fluoration de 1233xf en 1,1,1,2-tétrafluoro-2-chloropropane (HFC244bb), suivi d'une étape de déhydrochloration. Le produit 1233xf est préparé par trifluoration du précurseur chloré correspondant (CCl₂=CClCH₂Cl).

WO2008/054781 décrit une préparation de 1234yf par réaction du 2,3-dichloro-1,1,1-trifluoropropane (243db) en présence de HF, sur un catalyseur notamment Cr/Co 98/2. Les produits de la réaction comprennent du 1234yf et du 2-chloro-3,3,3-trifluoro-1-propène (1233xf), ce dernier produit étant majoritaire; les autres produits 1-chloro-3,3,3-trifluoro-1-propène (1233zd) ainsi que 245cb et 1234ze se forment aussi. Une température plus élevée favorise la production de l'isomère 1233zd. Le produit de départ 2,3-dichloro-1,1,1-trifluoropropane (243db) est indiqué comme étant obtenu par chloration du trifluoro-1-propène (TFP), sans autre précision.

WO2008/040969 et WO2008/075017 décrivent une préparation sensiblement similaire. Il est indiqué que la réaction procède par déhydrochloration du 243db en 1233 (tant xf que zd), suivi d'une réaction impliquant la formation de 1,1,1,2-tétrafluoro-2-chloropropane et la formation ultérieurement du 2,3,3,3-tétrafluoro-1-propène recherché par déhydrochloration. Le rapport HF:organiques est varié et il est indiqué que la réaction de déhydrochloration en 1233 (xf et zd) est favorisée par des ratios HF:organiques faibles tandis que la réaction de préparation du composé final recherché est favorisée par des ratios HF:organiques élevés. Le produit de départ 2,3-dichloro-1,1,1-trifluoropropane (243db) est indiqué comme étant obtenu par chloration du trifluoropropène ou du trifluoromethylpropène, sans autre précision.

Dans ces documents, aucun détail n'est donné sur la préparation du produit de départ, le 243db. Si les réactions de chloration, et notamment de chloration d'oléfine, sont connues, il n'y a pas de description précise dans l'état de la technique d'une étape de chloration du TFP en 243db. WO 2005108334 décrit l'addition d'un composé X₁X₂ sur du trifluoropropène, avec X₁ et X₂ qui peuvent être indépendamment du chlore. Il est indiqué encore l'addition de Cl₂ sur du TFP, comme première étape avant une réaction de fluoration. Cependant, les seuls exemples sur du TFP sont mis en oeuvre avec du brome et de l'iode, et en outre en présence de HF; le composé 2,3-dibromé ou 2,3-diiodé n'est ni isolé, ni préparé dans ce document.

La publication de R.N. Haszeldine, J.A.C.S. 2495-2504 (1951) décrit la synthèse de trifluorométhylacéthylène à partir de trifluoropropène en passant par le 2,3-dichloro-1,1,1-trifluoropropane. Les conditions opératoires ne sont pas indiquées à part un léger excès de chlore introduit. Il s'agit d'un essai en très petites quantités, dans des tubes scellés. Le rendement est situé entre 80 et 85%. Aucune information n'est donnée pour conduire le procédé à l'échelle industrielle.

La publication de M. Büchner et coll., Chemistry A European Journal 4, 1799-1809 (1998) décrit la préparation de 1,2-dihalo-3,3,3-trifluoropropane et de 2-chloro-3,3,3-trifluoro-propane. La température de réaction est de -30°C à -40°C. Cependant les quantités relatives de 3,3,3-trifluoro-1-propène et de chlore mis en réaction ne sont pas mentionnées, rien n'est indiqué non plus sur un possible recyclage ni sur les rendements et sélectivités obtenues. Rien dans ce documents n'indique comment rendre le procédé industriellement rentable.La réaction effective de chloration du TFP en 243db n'est donc pas décrite complètement dans la littérature.

Il existe un besoin d'un procédé de préparation de 1234yf à partir d'un produit de départ qui soit aisément accessible, et qui conduise au produit recherché avec une sélectivité élevée, et avantageusement un rendement et/ou une conversion élevé.

Il existe aussi un besoin d'un procédé de préparation de 243db, précurseur utile dans la préparation de 1234yf, à partir d'un produit de départ qui soit aisément accessible, et qui conduise au produit recherché avec une sélectivité élevée, et avantageusement un rendement et/ou une conversion élevée.

### RESUME DE L'INVENTION

L'invention fournit donc un procédé de préparation de 2,3-dichloro-1,1,1-trifluoropropane par chloration de 3,3,3-trifluoropropène à une pression supérieure à 2 bar mis en oeuvre en phase liquide et à une température comprise entre 20 et 200°C.

Selon un mode de réalisation, la pression est comprise entre 4 et 50 bar, de préférence entre 5 et 20 bar, avantageusement sous pression autogène.

Selon un mode de réalisation, ce procédé est mis en oeuvre en l'absence de catalyseur ajouté.

L'invention a encore pour objet un procédé de préparation de 2,3,3,3-tétrafluoro-1-propène comprenant les étapes suivantes: (i) chloration de 3,3,3-trifluoropropène en 2,3-dichloro-1,1,1-trifluoropropane par le procédé décrit ci-dessus de préparation du 243db; (ii) déhydrochloration du 2,3-dichloro-1,1,1-trifluoropropane obtenu à l'étape précédente en 2-chloro-3,3,3-trifluoro-1-propène; et (iii) fluoration du 2-chloro-3,3,3-trifluoro-1-propène obtenu à l'étape précédente en 2,3,3,3-tétrafluoro-1-propène.

Selon un mode de réalisation, dans ce procédé, l'étape (iii) est une fluoration catalytique directe.

Selon un mode de réalisation, dans ce procédé, l'étape (iii) comprend une première sous-étape (iiia) d'hydrofluoration en 1,1,1,2-tétrafluoro-2-chloropropane et une seconde sous-étape (iiib) de déhydrochloration en 2,3,3,3-tétrafluoro-1-propène.

Selon un mode de réalisation, dans ce procédé, les étapes (ii) et (iii) sont mises en oeuvre en phase gazeuse.

Selon un mode de réalisation, dans ce procédé, l'étape (ii) est mise en oeuvre en présence d'hydrogène.

Selon un mode de réalisation, dans ce procédé, la ou les réactions de déhydrochloration sont mises en oeuvre en présence de HF selon un ratio HF:produit de départ compris entre 1:1 et 5:1.

### DESCRIPTION DETAILLEE DE MODES DE REALISATION

L'invention utilise une étape de chloration de 3,3,3-trifluoropropène (1243zf) en 2,3-dichloro-1,1,1-trifluoropropane (243db).

Selon l'invention, cette étape est notamment mise en oeuvre sous une pression supérieure à 2 bar. Avantageusement, la pression est comprise entre 4 et 50 bar, notamment entre 5 et 20 bar. Il est possible de travailler à la pression autogène (env. 6 bar), en présence ou en l'absence de gaz ballast conduisant à une pression plus importante dans le milieu. Ce gaz inerte peut être notamment de l'hélium. Avantageusement, il n'y a pas de gaz ballast dans le système.

La réaction est mise en oeuvre en phase liquide. Sous pression autogène (environ 6 bars); le TFP est liquide. Sans vouloir être liée par une théorie, la demanderesse pense que le TFP (liquide) joue un rôle alors de solvant.

Le produit de réaction est liquide (notamment à température ambiante).

Le ratio molaire Cl₂ :TFP est compris entre 0,1 : 1 et 10:1, de préférence 0,2:1 à 5:1, avantageusement entre 0,5:1 et 1:1. L'intérêt d'opérer la réaction en quantité stoechiométrique est d'éviter de perdre le trifluoropropène en excès. Toutefois, compte tenu de l'exothermie de la réaction, il peut être utile de mettre en oeuvre la réaction avec un excès de TFP dans le réacteur, par exemple avec un ratio molaire Cl₂/TFP de 0,5. Le trifluoropropène sera alors recyclé.

La durée de réaction de la chloration est variable et peut être comprise entre 0,1 et 50 heures, de préférence entre 0,3 et 5 heures, avantageusement entre 0,5 et 3 heures.

La température de réaction est comprise entre 20°C (sensiblement température ambiante) et 200°C, de préférence entre 20°C et 150°C, avantageusement entre 40 et 100°C.

La réaction est une réaction exothermique (la chaleur de réaction est estimée à ΔH = -200 kJ/mol) . La température de réaction peut être contrôlée ou non. Si on souhaite contrôler la température de réaction, on peut utiliser un réacteur avec système de régulation (par ex. double-enveloppe) et en réglant la température de consigne. Celle-ci peut varier par exemple entre 20 et 100°C, avantageusement entre 30 et 80°C. L'exothermicité de la réaction conduit en général à une augmentation rapide de la température au début de la réaction, par exemple jusqu'à une température de 100°C, puis baisser ensuite jusqu'à la température de consigne du réacteur. L'utilisation de réacteur à double-enveloppe ou système équivalent est classique et bien connue pour l'homme du métier.

La réaction peut être mise en oeuvre en présence d'un catalyseur, par exemple à base de fer (sous forme métal ou FeCl₃). Avantageusement la réaction est mise en oeuvre en l'absence de catalyseur ajouté, ce qui simplifie la réaction.

Le produit de réaction peut être soumis à des étapes de séparation comme la distillation, le lavage, etc., de façon classique connue de l'homme du métier.

Selon un mode de réalisation, le produit brut est soumis à une filtration (après repos du produit brut de réaction). La filtration permet de récupérer un éventuel dépôt solide présent dans le milieu de réaction. Cette filtration est mise en oeuvre sur membrane, par exemple du type millipore®, avec une taille variable, par exemple entre 0,0.1 et 5 µm; de préférence entre 0,05 et 1 µm, avantageusement entre 0,1 et 0,5 µm. Ce temps de repos du produit brut de la réaction est compris entre 1 et 30 jours, de préférence entre 2 et 10 jours. La filtration peut être en mise en oeuvre avec tirage au vide. On obtient ainsi simplement le 243db, en éliminant la majeure partie du TFP (le tirage sous vide permet, par exemple à l'ambiante, de soutirer le TFP dont le point d'ébullition est inférieur à 0°C sous pression normale).

La réaction de chloration en phase liquide) sous pression peut donc être mise en oeuvre dans des conditions extrêmement simples (notamment pas de catalyseur, possibilité de travailler sous pression autogène) et est très efficace. La sélectivité en 243db est en général supérieure à 90%, de préférence supérieure à 95%, avantageusement supérieure à 98%, voire supérieure à 99%. La conversion du chlore en 243db est en général supérieure à 80%, avantageusement supérieure à 90%, voire supérieure à 95%.

Selon un autre aspect, l'invention est un procédé de préparation du 1234yf en trois étapes, dont la première est l'étape de chloration du TFP décrite ci-dessus.

Les réactions peuvent être en série, mises en oeuvre de façon continue ou non, en phase liquide ou gazeuse (la première étape étant mise en oeuvre en phase liquide), les produits de réaction étant envoyés dans l'étape suivante, éventuellement après avoir subi un traitement par exemple de séparation, si besoin est.

La seconde étape du procédé est une réaction de déhydrochloration du 2,3-dichloro-1,1,1-trifluoropropane (243db) obtenu à l'étape précédente en 2-chloro-3,3,3-trifluoro-1-propène (1233xf).

Les réactions de déhydrochloration sont mises en oeuvre de façon classique par l'homme du métier.

La réaction de déhydrochloration est de préférence mise en oeuvre avec un catalyseur de déhydrochloration. Ce catalyseur est par exemple un catalyseur à base d'un métal notamment d'un métal de transition ou un dérivé oxyde ou halogénure ou oxyhalogénure d'un tel métal. Des catalyseurs sont par exemple FeCl₃, oxyfluorure de chrome, Ni (incluant les treillis de Ni mesh), NiCl₂, CrF₃, et leurs mélanges, par exemple Ni-Cr/ALF3. D'autres catalyseurs possibles sont les catalyseurs supportés sur carbone, les catalyseurs à base d'antimoine, les catalyseurs à base d'aluminium (tel que AlF₃ et Al₂O₃ et oxyfluorure d'aluminium et alumine fluorée), de palladium, de platine, de rhodium et de ruthénium. On pourra se référer à la liste donnée dans le document US-P-5396000, colonne 1, ligne 50 à colonne 2, ligne 2 ou à la liste donnée dans WO 2007/056194, page 16, lignes 13-23. On pourra aussi utiliser les catalyseurs décrits dans WO 2008/040969, et notamment le Zn sur oxyde de chrome, traité par HF.

Cette étape de déhydrochloration est mise en oeuvre à une température comprise par exemple entre 220 et 400°C, de préférence entre 250 et 380°C.

Le temps de contact (rapport entre le volume de catalyseur et le flux total de la charge) est en général compris entre 0,1 et 100 secondes, de préférence entre 1 et 50 secondes.

Cette réaction peut être mise en oeuvre en phase gazeuse (éventuellement en présence d'un gaz inerte diluant) ou en phase liquide. On préférera la phase gazeuse.

Lorsque la réaction de déhydrochloration est mise en oeuvre en phase liquide, l'agent de déhydrochloration est notamment le KOH. Ce KOH est notamment disponible sous forme de solution aqueuse à une teneur en KOH de 10 à 50% en poids. La température est par exemple comprise entre -10 et 10°C. La chaleur peut être évacuée par un dispositif approprié. Le temps de réaction peut être compris entre 1 et 50 heures.

La réaction de déhydrochloration (notamment en phase gazeuse) peut être mise en oeuvre en l'absence de HF, ou en présence de HF. Si du HF est utilisé, il peut l'être selon un ratio molaire inférieur à 5:1 par rapport à la charge organique (243db), ou encore selon un ratio compris entre 5:1 et 15 : 1. Des ratios supérieurs, par exemple de 5:1 à 50:1, peuvent être envisagés. On préférera des ratios faibles, par exemple compris entre 1:1 et 5:1.

La présence de HF (selon des ratios variables) au cours de la réaction de déhydrochloration peut conduire, notamment lorsque la température est comprise entre 280 et 380°C à la production directe, outre le produit 1233xf, du produit final 1234yf recherché.

Il est aussi possible que du 1,1,1,2,2-pentafluoropentane soit produit; ce composé peut subir facilement une réaction de déhydrofluoration en le produit final 1234yf recherché; on pourra par exemple se référer à l'exemple 2 de WO 2008/040969 sur ce point.

On peut aussi prévoir des étapes de régénération du catalyseur, de façon connue.

La pression dans les différentes réactions, en phase gaz, peut être atmosphérique, ou inférieure ou supérieure à cette pression atmosphérique.

La réaction de déhydrochloration peut conduire à deux composés, isomères de position, le 2-chloro-3,3,3-trifluoro-1-propène (1233xf) et le 1-chloro-3,3,3-trifluoro-1-propène (1233zd). L'isomère non recherché, s'il est présent, peut être séparé à ce stade du mélange réactionnel. Il peut aussi rester dans le milieu réactionnel, et les produits de réaction ultérieurs peuvent éventuellement être séparés plus tard, si besoin.

De préférence, la réaction de déhydrochloration produit préférentiellement le composé 1233xf. De préférence, la sélectivité est supérieure à 80%, avantageusement supérieure à 90% et plus avantageusement supérieure à 95%. Selon le mode de réalisation dans lequel du 1234yf est produit concomitamment, la sélectivité est exprimée en tenant compte des deux composés 1233xf et 1234yf.

On peut aussi opérer en présence d'hydrogène pour améliorer la sélectivité en produit 1233xf.

La conversion est aussi, de préférence, très élevée, supérieure à 50%, avantageusement supérieure à 70% de préférence supérieure à 90%, voire supérieure à 95%.

En général, de l'hydrogène peut être injecté avec la charge de départ, par exemple en continu. Le ratio molaire H₂/charge départ peut varier dans de grandes mesures, notamment entre 0,3 et 30, notamment entre 0,5 et 20, avantageusement entre 1 et 10.

La mise en oeuvre des réactions de déhydrochloration en présence d'hydrogène permet donc une sélectivité élevée. La présence d' hydrogène permet aussi de réduire la production de lourds au cours de la réaction.

Un exemple de réaction de déhydrochloration du 243db en 1233xf (et 1234yf) est donné dans les documents cités supra, par exemple WO2008/054781, WO2008/040969 et WO2008/075017. On pourra se référer notamment au document WO 2008/040969, et en particulier pages 16 et 17, route A, et l'exemple 1.

La troisième étape du procédé de préparation du 1234yf est une réaction de fluoration du 2-chloro-3,3,3-trifluoro-1-propène (1233xf) obtenu à l'étape précédente en 2,3,3,3-tétrafluoro-1-propène, produit recherché.

Cette troisième étape peut comprendre la fluoration directe en présence de HF, sur un catalyseur, en phase gazeuse. Les catalyseurs de fluoration susceptibles d'être utilisés sont par exemple oxyfluorure de chrome, fluorure et oxyfluorure d'aluminium, catalyseur supporté contenant un métal tel que Cr, Zn, Ti, V, Zr, Mo, Ge, Sn, Pb. Les températures, pressions et temps de contact sont aisément déterminés par l'homme du métier.

Un tel procédé est décrit, en référence au composé 1233zd qui conduit au composé 1234ze, dans la demande EP-A-1067106, (notamment exemple 1), à laquelle il est fait référence. L'application du procédé au produit de départ 1233xf conduira à la formation du produit 1234yf. La formation concomitante du produit d'hydrofluoration est possible, bien que non recherché. Le produit de réaction saturé peut alors être déhydrohalogéné, dans des conditions similaires à celles de la seconde étape du présent procédé, pour conduire au produit recherché.

Cette troisième étape peut aussi comprendre deux sous-étapes, une première sous-étape de formation du produit 1,1,1,2-tétrafluoro-2-chloropropane (244bb), puis une seconde sous-étape de déhydrochloration de ce produit en le 1234yf recherché.

La première sous-étape comprend l'hydrofluoration du 1233xf, qui peut être mise en oeuvre dans des conditions connues. Par exemple, la réaction peut se faire en phase gaz ou liquide, sur un catalyseur qui peut être un acide de Lewis, ou un halogénure d'un métal, comme par exemple SbCl₅, SbCl₃, SbF₅, SnCl₄, TiCl₄, FeCl₃, etc. La température peut être comprise entre 50°C et 400°C, par exemple de 150°C à 300°C. Le temps de contact est déterminé en fonction des conversion et sélectivité recherchés. La première sous-étape est notamment décrite dans le document WO 2007/079431, page 8, ligne 17 à page 10, ligne 16 et exemples 5A, 5B et 6, auquel il fait référence. On pourra encore aussi se référer au document WO 2008/040969, pages 16 & 17. Dans cette réaction, le ratio HF:1233xf est de préférence supérieur à 5:1, et en général compris entre 5:1 et 50:1, ou encore entre 15:1 et 30:1, afin de favoriser la réaction de fluoration.

La seconde sous-étape est une étape de déhydrochloration, qui est mise en oeuvre dans les conditions décrites ci-dessus pour la réaction de déhydrochloration de la seconde étape du procédé de préparation du 1234yf. Pour cette seconde sous-étape on pourra se référer au document WO 2007/079431, page 10, ligne 18 à page 12, ligne 14 et exemple 6, auquel il fait référence. On se référera aussi au document WO 2008/040969, pages 16 & 17.

Lorsque la seconde étape du procédé conduit à la formation du composé 1233zd, ce composé peut éventuellement être soumis aux conditions de cette troisième étape lorsqu'elle est mise en oeuvre avec les deux sous-étapes. Dans un tel cas, la première sous-étape conduit au composé CF₃-CHF-CH₂Cl (244eb), qui est à son tour déhydrochloré en le produit recherché. Cette dernière réaction est aussi couverte par la description générique faite dans le document WO 2007/056194 déjà mentionné supra.

Les réactions sont mises en oeuvre dans un ou plusieurs réacteurs dédiés aux réactions impliquant des halogènes. De tels réacteurs sont connus de l'homme du métier, et peuvent comprendre des revêtements intérieurs à base par exemple de Hastelloy®, Inconel®, Monel® ou de fluoropolymères. Le réacteur peut aussi comprendre des moyens d'échange de chaleur, si besoin.

L'alimentation en réactifs se fait en général en continu, mais peut être étagée le cas échéant. Les points pour les éventuel(s) séparation(s) et/ou recyclage(s) sont variables, en tête de procédé ou à des niveaux intermédiaires.

### EXEMPLES

Les exemples suivants illustrent l'invention sans la limiter (les pressions sont en bars).

### Exemple 1.

L'appareillage utilisé est constitué d'un autoclave avec double enveloppe d'une capacité de 1 litre, en inox 316L, agité. Celui-ci est équipé d'une mesure de pression et de température. Des piquages sur la tête de l'autoclave permettent l'introduction des réactifs et le dégazage. Un test d'étanchéité est effectué après avoir introduit le catalyseur solide, à savoir un coupon métallique de 46 g. de fer préalablement décapé dans une solution d'acide chlorhydrique. 1,098 mol (78 g) de chlore sont introduits dans le réacteur préalablement placé sous vide et refroidi dans l'azote liquide. 1,098 mol (105,5 g) de trifluoropropène sont ensuite ajoutés. La pression autogène est d'environ 6 bars. Le réacteur est pressurisé jusqu'à 10 bars absolus à l'aide d'hélium. Le milieu réactionnel est laissé sous agitation pendant 4 heures. Le réacteur est ensuite lentement dépressurisé. On récupère ainsi 163,4 g d'un liquide trouble de coloration légèrement jaune au fond du réacteur. La nature de ce liquide est analysée à l'aide d'un chromatographe phase gaz équipé d'une colonne Carbopack® B1%SP1000. Le liquide récupéré est constitué majoritairement de 243db (98,0 mol%) et de TFP (1,4 mol%) résiduel (dissous dans le 243db). Des impuretés en très faible quantité issues d'un, mécanisme de réaction radicalaire ont toutefois pu être identifiées : CF₃-CCl=CH₂, CF₃-CH₂-CH₂Cl, CF₃-CHCl-CH₂Cl. La phase gaz a également été analysée et contient 22% de TFP et 77,4% de 243db. Compte tenu du volume du ciel du réacteur (800 ml), de la pression de 6 bars et de la température au dégazage, on calcule 0,038 mol de TFP dans la phase gaz et 0,134 mole de 243db. La conversion du TFP est donc de 95,3% et la sélectivité en 243db supérieure à 99%.

Une filtration sur membrane millipore 0,22 µm permet d'obtenir un liquide transparent et d'éliminer en même temps une partie du trifluoropropène résiduel. La solution de 243 db résultante a une pureté de 99,05% avec 0,7% de trifluoropropène.

## Revendications

1. Procédé de préparation de 2,3-dichloro-1,1,1-trifluoropropane par chloration de 3,3,3-trifluoropropène à une pression supérieure à 2 bar, mis en oeuvre en phase liquide, à une température comprise entre 20 et 200 °C.

2. Procédé selon la revendication 1, dans lequel la pression est comprise entre 4 et 50 bar, de préférence entre 5 et 20 bar, avantageusement sous pression autogène.

3. Procédé selon la revendication 1 ou 2, mis en oeuvre en l'absence de catalyseur ajouté.

4. Procédé de préparation de 2,.3,3,3-tétrafluoro-1-propène comprenant les étapes suivantes:
(i) chloration de 3,3,3-trifluoropropène en 2,3-dichloro-1,1,1-trifluoropropane par le procédé selon l'une des revendications précédente ;
(ii) déhydrochloration du 2,3-dichloro-l-1,1,1-trifluoropropane obtenu à l'étape précédente en 2-chloro-3,3,3-trifluoro-1-propène;
(iii) fluoration du 2-chloro-3,3,3-trifluoro-1-propène obtenu à l'étape précédente en 2,3,3,3-tétrafluoro-1-propène.

5. Procédé selon la revendication 4, dans lequel l'étape (iii) est une fluoration catalytique directe.

6. Procédé selon la revendication 4, dans lequel l'étape (iii) comprend une première sous-étape (iiia) d'hydrofluoration en 1,1,1,2-tétrafluoro-2-chloropropane et une seconde, sous-étape (iiib) de déhydrochloration en 2,3,3,3-tétrafluoro-1-propène.

7. Procédé selon l'une des revendications 4 à 6, dans lequel les étapes (ii) et (iii) sont mises en oeuvre en phase gazeuse.

8. Procédé selon l'une des revendications 4 à 7, dans lequel l'étape (ii) est mise en oeuvre en présence d'hydrogène.

9. Procédé selon l'une des revendications 4 à 8, dans lequel la ou les réactions de déhydrochloration sont mises en oeuvre en présence de HF selon un ratio HF:produit de départ compris entre 1:1 et 5:1.

## Patentansprüche

1. Verfahren zur Herstellung von 2,3-Dichlor-1,1,1-trifluorpropan durch Chlorierung von 3,3,3-Trifluoropropen bei einem Druck von mehr als 2 bar, wobei die Durchführung in der Flüssigphase bei einer Temperatur im Bereich von 20 bis 200 °C erfolgt.

2. Verfahren nach Anspruch 1, wobei der Druck im Bereich von 4 bis 50 bar liegt, vorzugsweise von 5 bis 20 bar, vorteilhafterweise unter autogenem Druck.

3. Verfahren nach Anspruch 1 oder 2, wobei die Durchführung ohne zugesetzten Katalysator erfolgt.

4. Verfahren zur Herstellung von 2,3,3,3-Tetrafluor-1-propen, die folgenden Schritte umfassend:
(i) Chlorierung von 3,3,3-Trifluorpropen zu 2,3-Dichlor-1,1,1-trifluorpropan mittels des Verfahrens nach einem der vorhergehenden Ansprüche;
(ii) Dehydrochlorierung des 2,3-Dichlor-1,1,1-trifluorpropans, das im vorhergehenden Schritt erhalten wurde, zu 2-Chlor-3,3,3-trifluor-1-propen;
(iii) Fluorierung des 2-Chlor-3,3,3-trifluor-1-propens, das im vorhergehenden Schritt erhalten wurde, zu 2,3,3,3-Tetrafluor-1-propen.

5. Verfahren nach Anspruch 4, wobei der Schritt (iii) eine direkte katalytische Fluorierung darstellt.

6. Verfahren nach Anspruch 4, wobei der Schritt (iii) einen ersten Teilschritt (iiia) der Hydrofluorierung zu 1,1,1,2-Tetrafluor-2-chlorpropan und einen zweiten Teilschritt (iiib) der Dehydrochlorierung zu 2,3,3,3-Tetrafluor-1-propen umfasst.

7. Verfahren nach einem der Ansprüche 4 bis 6, wobei die Schritte (ii) und (iii) in der Gasphase durchgeführt werden.

8. Verfahren nach einem der Ansprüche 4 bis 7, wobei der Schritt (ii) in Gegenwart von Wasserstoff durchgeführt wird.

9. Verfahren nach einem der Ansprüche 4 bis 8, wobei die Dehydrochlorierungsreaktion(en) in Gegenwart von HF gemäß einem HF:Edukt-Verhältnis im Bereich von 1:1 bis 5:1 durchgeführt wird/werden.

## Claims

1. A process for the preparation of 2,3-dichloro-1,1,1-trifluoropropane by chlorination of 3,3,3-trifluoropropene at a pressure of more than 2 bar, carried out in the liquid phase, at a temperature in the range 20 to 200°C.

2. The process as claimed in claim 1, in which the pressure is in the range 4 to 50 bar, preferably in the range 5 to 20 bar, advantageously under autogenous pressure.

3. The process as claimed in claim 1 or claim 2, carried out in the absence of an added catalyst.

4. A process for the preparation of 2,3,3,3-tetrafluoro-1-propene, comprising the following steps:
(i) chlorination of 3,3,3-trifluoropropene to form 2,3-dichloro-1,1,1-trifluoropropane by the process as claimed in one of the preceding claims;
(ii) dehydrochlorination of the 2,3-dichloro-1,1,1-trifluoropropane obtained in the preceding step to form 2-chloro-3,3,3-trifluoro-1-propene ;
(iii) fluorination of the 2-chloro-3,3,3-trifluoro-1-propene obtained in the preceding step to form 2,3,3,3-tetrafluoro-1-propene.

5. The process as claimed in claim 4, in which step (iii) is a direct catalytic fluorination.

6. The process as claimed in claim 4, in which step (iii) comprises a first sub-step (iiia) of hydrofluorination to form 1,1,1,2-tetrafluoro-2-chloropropane and a second sub-step (iiib) of dehydrochlorination to form 2,3,3,3-tetrafluoro-l-propene.

7. The process as claimed in one of claims 4 to 6, in which steps (ii) and (iii) are carried out in the gaseous phase.

8. The process as claimed in one of claims 4 to 7, in which step (ii) is carried out in the presence of hydrogen.

9. The process as claimed in one of claims 4 to 8, in which the dehydrochlorination reaction or reactions are carried out in the presence of HF in a HF: starting product ratio in the range 1:1 to 5:1.
